Europäisches Patentamt

European Patent Office

Office européen des brevets

(11) Publication number: **0 109 702**
**B1**

(12) **EUROPEAN PATENT SPECIFICATION**

(45) Date of publication of patent specification: 09.03.88

(21) Application number: 83201557.2

(22) Date of filing: 31.10.83

(51) Int. Cl.⁴: **C 07 C 1/04, B 01 J 23/74,**
**B 01 J 23/86**

(54) Process for the preparation of hydrocarbons.

(30) Priority: 22.11.82 NL 8204526

(43) Date of publication of application:
30.05.84 Bulletin 84/22

(45) Publication of the grant of the patent:
09.03.88 Bulletin 88/10

(84) Designated Contracting States:
AT BE DE FR IT NL SE

(56) References cited:
GB-A-2 077 289

(73) Proprietor: SHELL INTERNATIONALE
RESEARCH MAATSCHAPPIJ B.V.
Carel van Bylandtlaan 30
NL-2596 HR Den Haag (NL)

(72) Inventor: Minderhoud, Johannes Kornelis
Badhuisweg 3
NL-1031 CM Amsterdam (NL)
Inventor: Post, Martin Franciscus Maria
Badhuisweg 3
NL-1031 CM Amsterdam (NL)
Inventor: Sie, Swan Tiong
Badhuisweg 3
NL-1031 CM Amsterdam (NL)

(74) Representative: Aalbers, Onno et al
P.O. Box 302
NL-2501 CH The Hague (NL)

Courier Press, Leamington Spa, England.

# 0 109 702

**Description**

The invention relates to a process for the preparation of hydrocarbons by catalytic reaction of carbon monoxide with hydrogen.

The preparation of hydrocarbons from a $H_2/CO$ mixture by contacting this mixture at elevated temperature and pressure with a catalyst is known in the literature as Fischer-Tropsch hydrocarbon synthesis. Catalysts often used for this purpose contain iron or cobalt together with one or more promoters and/or a carrier material. The conventional techniques of preparing Fischer-Tropsch catalysts are the precipitation route, the melting route and the impregnation route. Of these techniques the impregnation route is much to be preferred since this route is not so costly and/or time consuming, produces better reproducible results and generally yields materials with better catalytic properties. Briefly, the impregnation route involves contacting a porous carrier in the presence of a liquid once or several times with an iron or cobalt compound and, if desired, with one or more compounds of the appropriate promoters, followed by removal of the liquid and calcination and reduction of the composition obtained. The preparation of catalysts which, in addition to iron or cobalt, contain one or more promoters may be carried out both by co-impregnation and by separate impregnation. In the case of co-impregnation the porous carrier is contacted once or several times with both an iron or cobalt compound and one or more compounds of the relevant promoters. In the case of separate impregnation the porous carrier is first contacted with an iron or cobalt compound or with one or more compounds of the relevant promoters and, after calcination of the composition thus obtained, the latter is contacted with one or more compounds of the relevant promoters or with an iron or cobalt compound, respectively, followed by calcination and reduction of the composition. For preparing Fischer-Tropsch catalysts comprising cobalt supported on a carrier an attractive method was recently found which can be carried out in a simple manner and yields catalysts of very good properties. In this method the deposition of the cobalt on the carrier is carried out by kneading instead of impregnation. The catalyst preparation by kneading is similar to the preparation by impregnation carried out by contacting a porous carrier with one or more compounds of the catalytically active metals in the presence of a liquid, followed by the removal of the liquid and calcination of the composition, but before and/or during the removal of the liquid the composition is subjected to an intensive mechanical treatment such as pressing, squeezing or wringing, which generally has as a result that a substantial decrease of the particle size of the carrier material occurs and that the composition takes on the consistency of a paste. Generally several hours' kneading in a suitable kneading machine is sufficient to achieve the desired homogeneous dispersion of the components over the mixture. The intensive mechanical treatment in which a substantial decrease of the particle size of the carrier material occurs, forms the principle difference between the kneading route and the impregnation route. It is true that in the preparation of a catalyst by impregnation a stage may be passed in which the composition contains an amount of liquid corresponding to that present in the above-mentioned paste and that—e.g. by stirring—some mechanical energy may be supplied to the composition, but as a rule the particle size of the carrier material remains substantially unchanged in the catalyst preparation by impregnation. Hereinafter, whenever mention is made of the preparation of catalysts by kneading, this should be taken to be the above-described preparation route in which at least the cobalt has been deposited on the carrier by kneading. Any promoters present on the carrier may also have been deposited on the carrier by kneading or impregnation.

The composition of the product obtained in the Fischer-Tropsch hydrocarbon synthesis by using catalysts prepared by impregnation and/or kneading is largely dependent on the catalytically active metal which is present on the catalyst. The use of a cobalt catalyst prepared by impregnation and/or kneading results in a product which consists mainly of unbranched paraffins. The use of an iron catalyst prepared by impregnation results in a product which contains a considerable quantity of olefins and oxygen-containing organic compounds. With a view to the composition of the product obtained, for the preparation of products suitable for use as motor fuels, preference is given to the use of a cobalt catalyst. Especially catalysts which, in addition to cobalt, contain silica and in which the quantity of cobalt is 10—40 pbw per 100 pbw silica, are very suitable for the present purpose.

From GB—A—2077289 a process is known for the preparation of hydrocarbons by contacting a feed which comprises hydrogen and carbon monoxide at elevated temperature and pressure with a catalyst which comprises 10 to 40 pbw cobalt and 0.25 to 5 pbw of a promoter chosen from the group formed by zirconium, titanium or chromium per 100 pbw silica and which has been prepared by depositing cobalt and the promoter on a silica carrier by impregnation and calcining and reducing the composition obtained. If the feed comprising the hydrogen and carbon monoxide has an $H_2/CO$ molar ratio of less than 1.5, water has to be added to said feed and the above-mentioned cobalt-containing catalyst must be used in combination with a CO-shift catalyst to convert carbon monoxide and water into carbon dioxide and hydrogen, thus raising the $H_2/CO$ molar ratio.

An important parameter in the preparation of hydrocarbons from $H_2/CO$ mixtures is the $C_3^+$ selectivity of the catalyst used. According as this $C_3^+$ selectivity is higher, a larger percentage of the hydrocarbon mixture obtained will consist of valuable $C_3^+$ hydrocarbons and consequently a smaller percentage will consist of $C_2^-$ hydrocarbons as by-products. Although the afore-mentioned catalysts containing 10—40

2

pbw cobalt per 100 pbw silica do already have a $C_3^+$ selectivity of about 80%, the need is nevertheless felt to boost the selectivity of these catalysts and so to reduce the formation of undesirable $C_2^-$ by-products.

An investigation carried out into this subject revealed that the $C_3^+$ selectivity of catalysts containing 10—40 pbw cobalt per 100 pbw silica can be considerably enhanced by using a feed which contains steam, provided that the following conditions are met:

a) the catalyst must contain 0.1—150 pbw of a promoter chosen from the group formed by zirconium, titanium or chromium per 100 pbw silica,

b) the cobalt and the promoter must have been deposited on the silica by impregnation and/or kneading, and

c) the quantity of steam present in the feed must be 10—40% v, calculated on the $H_2/CO/H_2O$ mixture.

The following may be remarked on points a)—c).

Experiments using non-promoted $Co/SiO_2$ catalysts prepared by kneading have shown that the presence of steam in the feed has no effect on the $C_3^+$ selectivity of the catalysts. Experiments using $Co/Zr/SiO_2$ catalysts substantially prepared by precipitation showed that neither for these catalysts did the presence of steam in the feed have an effect on the $C_3^+$ selectivity. Experiments using $Co/Zr/SiO_2$ catalysts prepared by impregnation and/or kneading and using feeds of various steam contents showed that significant enhancement of the $C_3^+$ selectivity of these catalysts can only be achieved when the quantity of steam is at least 10 and at most 40% v, calculated on the $H_2/CO/H_2O$ mixture. The use of feeds with lower or higher steam contents (e.g. 5 or 50% v) yields an insufficient increase of the $C_3^+$ selectivity. When feeds with very high steam contents (e.g. 60% v) are used, the $C_3^+$ selectivity will be even lower than in steam-free operations.

Attempts at utilizing the afore-mentioned find for raising the $C_3^+$ selectivity of the closely related iron catalysts have remained unsuccessful. Experiments using promoted $Fe/SiO_2$ catalysts prepared by impregnation have shown that the use both of feeds with low steam contents (e.g. 5% v) and of feeds with higher steam contents (e.g. 30% v) results in a very severe drop of $C_3^+$ selectivity. Furthermore, contrary to what is seen for the cobalt catalysts, the presence of steam in the feed has quite an adverse effect on the activity of the iron catalysts.

The present patent application therefore relates to a process for the preparation of hydrocarbons by catalytic reaction of carbon monoxide with hydrogen, in which a feed which contains hydrogen, carbon monoxide and steam and in which the quantity of steam present is 10—40% v, calculated on the $H_2/CO/H_2O$ mixture is contacted at elevated temperature and pressure with a catalyst which comprises 5—40 pbw cobalt and 0.1—150 pbw of a promoter chosen from the group formed by zirconium, titanium or chromium per 100 pbw of silica and which has been prepared by depositing cobalt and the promoter on a silica carrier by impregnation and/or kneading and calcining and reducing the composition obtained, with the proviso that the catalyst does not contain an additional CO-shift catalyst.

The catalysts used in the process according to the invention may be prepared either exclusively by impregnation, or exclusively by kneading, or by a combination of kneading and impregnation.

If the preparation of the catalysts is carried out exclusively by impregnation, both co-impregnation and separate impregnation are eligible. With co-impregnation the silica carrier is contacted once or several times with a Co compound and a Zr, Ti or Cr compound. With separate impregnation optionally either first the Co and, after calcination of the composition, the Zr, Ti or Cr or first the Zr, Ti or Cr and, after calcination of the composition, the Co may be deposited on the silica carrier. Both the deposition of the Co and the deposition of the Zr, Ti or Cr may be carried out in one or more steps. After the last impregnation step of the co-impregnation the metal-containing composition should be calcined. If the catalyst preparation is carried out by separate impregnation, the metal-containing composition should be calcined both after the last impregnation step in which the cobalt has been deposited on the silica and after the last impregnation step in which the promoter has been deposited on the silica. If the co-impregnation or the separate impregnation are carried out in more than one step, the metal-containing composition obtained is preferably calcined after each impregnation step.

In the preparation of catalysts by a combination of kneading and impregnation first cobalt is deposited on the carrier by kneading, and then the promoter by impregnation. After calcination of the paste the composition obtained is impregnated with a compound of the promoter and calcined again.

If the preparation of the catalysts is carried out exclusively by kneading the starting material may very suitably be a mixture which, in addition to silica, a liquid and a Co compound, contains a Zr, Ti or Cr compound.

The amount of liquid which may be used in the preparation of catalysts by impregnation may vary within wide limits. The impregnation is preferably carried out as what is called a "dry impregnation", which means that a quantity of liquid is applied, the volume of which substantially corresponds with the pore volume of the carrier. The amount of liquid which may be used in the preparation of catalysts by kneading may also vary within wide limits. Amounts of liquid which are smaller, equal to or larger than the pore volume of the carrier come into consideration, provided that during kneading such an amount of liquid is present that under the influence of the intensive mechanical treatment the carrier material, together with the metal compound, can yield a composition with the desired paste-like consistency. A possible excess of liquid may be removed from the composition by evaporation before or during kneading. Preferably the quantity of liquid used in the kneading has a volume which corresponds to 110—190% of the pore volume of the carrier material.

In the deposition of a metal by impregnation as well as in the deposition of a metal by kneading, the carrier material is first of all contacted with a compound of the metal concerned in the presence of a liquid. As metal compounds both organic and inorganic compounds are eligible. As liquids both organic and inorganic liquids may be applied. In the catalyst preparation by impregnation as well as in the catalyst preparation by kneading preference is given to contacting the porous carrier with a solution of the metal compound concerned in a solvent. Examples of suitable aqueous solutions of zirconium compounds are solutions of zirconium nitrate or of zirconium chloride in water. Examples of suitable non-aqueous solutions of zirconium and titanium compounds are solutions of zirconium tetrapropoxide in a mixture of benzene and propanol and solutions of tetraisopropyl orthotitanate in a mixture of isopropanol and acetyl acetone. For the deposition of cobalt on the carrier preference is given to the use of a solution of an inorganic cobalt compound in water. Cobalt compounds suitable for the purpose are cobalt nitrate, cobalt carbonate, cobalt chloride and cobalt sulphate.

The calcination(s) to be carried out in the present catalyst preparations is (are) preferably carried out at a temperature between 350 and 700°C. After the last calcination the Co and Zr, Ti or Cr-containing composition should be reduced. This reduction is preferably carried out at a temperature between 200 and 350°C.

The quantities of promoter present on the catalyst is more than 0.1 pbw per 100 pbw silica. Depending on the method of preparation chosen, the catalysts may contain up to 150 pbw per 100 pbw silica. If the catalyst preparation is carried out by separate impregnation in which first the promoter and then the cobalt is deposited on the silica, preference is given to the use of catalysts containing 2—100 and in particular 2—20 pbw promoter per 100 pbw silica. If the catalyst preparation is carried out by co-impregnation, by separate impregnation in which first the cobalt and then the promoter is deposited on the silica, by kneading or by a combination of kneading and impregnation, preference is given to catalysts containing 0.1—5 pbw and in particular 0.25—2 pbw, promoter per 100 pbw silica.

In the process according to the invention preference is given to the use of catalysts which have been prepared either by separate impregnation in which first the promoter and then the cobalt is deposited on the silica, or by kneading or a combination of kneading and impregnation. As regards the promoter present on the catalysts, preference is given to the use of zirconium.

The process according to the invention is preferably carried out at a temperature of 125—350°C and in particular of 175—275°C and a pressure of 5—150 bar and in particular of 10—100 bar. In the process according to the invention the starting material is a $H_2$, CO and $H_2O$-containing feed which preferably has a $H_2/CO$ molar ratio higher than 1.75.

The process according to the invention may very suitably be carried out as an independent process in which a $H_2$ and CO-containing feed, after the addition of steam, is converted in one step into a hydrocarbon mixture. A very suitable feed may in this case be produced by the addition of steam to a $H_2/CO$ mixture obtained by gasification of a carbon-containing material such as coal.

The process according to the invention may also very suitably be carried out as part of a multi-step process for the conversion of a $H_2$ and CO-containing feed. This case offers three options, viz.

A) The process is used as second step in two two-step processes.

B) The process is used as the first step of a two-step process.

C) A combination of the processes mentioned under A) and B), with the process according to the invention being used as second step in two three-step processes.

Each one of these multi-step processes will be further explained hereinafter.

In the processes mentioned under A) two embodiments may be distinguished, depending on whether the first step is intended for the preparation of a $H_2/CO$ mixture with the desired steam content by catalytic conversion of light hydrocarbons (embodiment A1) or whether the first step is intended for the preparation of hydrocarbons and/or oxygen-containing organic compounds by catalytic conversion of a $H_2/CO$ mixture (embodiment A2).

The process mentioned under A1) bears upon the fact that a very attractive method of preparing $H_2/CO$ mixtures consists in steam reforming light hydrocarbons, such as methane. In this method the light hydrocarbons together with steam are contacted at high temperature and pressure with a catalyst. Catalysts often used for the purpose are nickel-containing catalysts. It is common practice in order to protect the catalysts from excessive coke deposition to carry out this reaction in the presence of an excess of steam, viz. considerably more steam than stoichiometrically required for the chemical conversion of the hydrocarbons into a $H_2/CO$ mixture. The excess to be used of steam which will ultimately find its way into the ready $H_2/CO$ mixture may vary within wide limits. A correct choice of the excess of steam used in the first step will result in the production of a $H_2/CO$ mixture which contains the required quantity of steam, so that without any further addition of steam this $H_2/CO/H_2O$ mixture can be used as the feed for the process according to the invention when used as second step.

In the process mentioned under A2) a $H_2/CO$ mixture is contacted in the first step with a catalyst containing one or more metal components having catalytic activity for the conversion of a $H_2/CO$ mixture into hydrocarbons and/or oxygen-containing organic compounds, and unconverted hydrogen and carbon monoxide present in the reaction product of the first step—together with other components of that reaction product, if desired—after the addition of steam, are used as the feed for the process according to the invention. Depending on the nature of the catalyst chosen in the first step either substantially aromatic hydrocarbons, or substantially paraffinic hydrocarbons, or substantially oxygen-containing compounds may be prepared in this step. If it is the object in the first step to prepare substantially aromatic hydrocarbons, then use may quite suitably be made of a catalyst mixture containing either a methanol or dimethyl ether synthesis catalyst or a $Fe/Mg/Al_2O_3$ or $Fe/Cr/SiO_2$ catalyst prepared by impregnation together with a cristalline metal silicate which is characterized in that after one hour's calcination in air at 500°C it has the following properties:

a) an X-ray powder diffraction pattern in which the strongest lines are the lines mentioned in Table A

TABLE A

$d(Å)$
11.1 ±0.2
10.0 ±0.2
3.84±0.07
3.72±0.06, and

b) in the formula which represents the composition of the silicate expressed in moles of the oxides and in which, in addition to $SiO_2$, one or more oxides of a trivalent metal M, chosen from the group formed by aluminium, iron, gallium, rhodium, chromium and scandium are present, the $SiO_2/M_2O_3$ molar ratio is higher than 10.

If it is the object in the first step of the two-step process mentioned under A2) to prepare substantially paraffinic hydrocarbons then use may very suitably be made of the above-mentioned $Fe/Mg/Al_2O_3$ or $Fe/Cr/SiO_2$ catalysts prepared by impregnation. If the first step of the two-step process mentioned under A2) is carried out with the object of preparing oxygen-containing organic compounds, then use may very suitably be made of a methanol or dimethyl ether synthesis catalyst. Methanol synthesis catalysts suitable for use in the first step of the two-step process mentioned under A2) are $ZnO/Cr_2O_3$ and $Cu/ZnO/Cr_2O_3$ catalysts. A dimethyl ether synthesis catalyst suitable for use in the first step of the two-step process mentioned under A2) is a mixture of gamma-$Al_2O_3$ and the $Cu/ZnO/Cr_2O_3$ methanol synthesis catalyst mentioned hereinbefore.

In the two-step process mentioned under B) as well as in the three-step processes mentioned under C) the fact is utilized that the high-boiling part of the product obtained in the process according to the invention can be converted in a high yield into middle distillates using a catalytic hydrotreatment. In the present patent application the term "middle distillates" is used to designate hydrocarbon mixtures whose boiling range corresponds substantially with that of the kerosine and gas oil fractions obtained in the conventional atmospheric distillation of crude mineral oil. Said distillation is used to separate from crude mineral oil one or more gasoline fractions having a boiling range between 30 and 200°C, one or more kerosine fractions having a boiling range between 140 and 300°C and one or more gas oil fractions having a boiling range between 180 and 370°C, successively.

The two-step process mentioned under B) comprises carrying out the process according to the invention as the first step, followed by a catalytic hydrotreatment as the second step. The three-step processes mentioned under C) comprise carrying out the two-step processes mentioned under A1) and A2), with the process according to the invention forming the second steps followed by a catalytic hydrotreatment as the third step. The feed chosen for the catalytic hydrotreatment is at least the part of the reaction product of the process according to the invention whose initial boiling point lies above the final boiling point of the heaviest middle distillate desired as final product. The hydrotreatment which is characterized by a very low hydrogen consumption yields middle distillates with a considerably lower pour point than that of those obtained in the direct Fischer-Tropsch conversion of a $H_2/CO$ mixture have. Catalysts very suitable for carrying out the catalytic hydrotreatment are those containing one or more noble metals of Group VIII supported on a carrier, and particularly suitable is a catalyst containing platinum supported on a carrier 13—15% w of which consists of alumina, the rest of silica.

The invention is now illustrated with the aid of the following example.

Example

Eight catalysts (catalysts 1—8) were prepared. Catalyst 1 contained 25 pbw iron, 1.25 pbw copper and 2 pbw potassium per 100 pbw silica. Catalyst 2 contained 25 pbw cobalt per 100 pbw silica. Catalysts 3—7, in addition to 25 pbw cobalt per 100 pbw silica, contained varying quantities of zirconium. Catalyst 8 contained 25 pbw cobalt and 0.9 pbw titanium per 100 pbw silica. All the catalysts were prepared starting from the same silica carrier. All the impregnations were carried out as dry impregnations. After each impregnation step the compositions obtained were dried at 120°C and calcined in air at 500°C. After the last calcination step the compositions were reduced in hydrogen; catalyst 1 at 280°C and the other catalysts at 250°C. Further information concerning the preparation of the catalysts is given hereinafter.

5

Catalyst 1
One-step impregnation of the silica carrier with an aqueous solution containing both iron nitrate, copper nitrate and potassium nitrate.

Catalyst 2
Kneading of a mixture comprising the silica carrier, water and cobalt nitrate. The mixture contained a quantity of water corresponding with 150% v of the pore volume of the carrier. After 3.5 hours of kneading the kneaded mass was dried at 120°C and calcined in air at 500°C.

Catalyst 3
A boiling solution of cobalt nitrate in water was added with stirring to a boiling solution of soda in water. Then, to the mixture thus obtained soda and the silica carrier were added in succession. After filtration of the mixture the filter cake was washed, dried at 120°C and calcined in air at 500°C. The composition thus obtained was impregnated in one step with an aqueous solution of zirconium nitrate. Catalyst 3 contained 0.9 pbw zirconium per 100 pbw silica.

Catalyst 4
Impregnation of the silica carrier first in one step with an aqueous solution of cobalt nitrate and then in one step with an aqueous solution of zirconium nitrate. Catalyst 4 contained 0.9 pbw zirconium per 100 pbw silica.

Catalyst 5
The Co/SiO$_2$ composition the preparation of which has been described under catalyst 2 was impregnated in one step with an aqueous solution of zirconium nitrate. Catalyst 5 contained 0.9 pbw zirconium per 100 pbw silica.

Catalyst 6 and 7
One-step or multi-step impregnation of the silica carrier with a solution of zirconium tetrapropoxide in a mixture of propanol and benzene, followed by impregnation in one-step with an aqueous solution of cobalt nitrate. In the preparation of catalysts 6 and 7 the impregnation of the silica carrier with the zirconium tetrapropoxide solution was carried out in one and three steps, respectively. Per 100 pbw silica catalysts 6 and 7 contained 6 and 18 pbw zirconium, respectively.

Catalyst 8
Impregnation of the silica carrier, first in one step with a solution of tetraisopropyl orthotitanate in a mixture of isopropanol and acetyl acetone and then in one step with an aqueous solution of cobalt nitrate. Catalyst 8 contained 0.9 pbw titanium per 100 pbw silica.
The cobalt loads and surface areas of the catalysts 4—8 are given in Table B.
Of the above-described catalysts 1—8 only catalysts 4—8 are suitable to be used in the process according to the invention. The other catalysts fall outside the scope of the invention. They have been included in the patent application for comparison.
Catalysts 1—8 were used in thirty experiments (Experiments 1—30) in the preparation of hydrocarbons from mixtures of carbon monoxide and hydrogen. The experiments were carried out in a 50 ml reactor containing a fixed catalyst bed of 7.5 ml volume.
The conditions under which the experiments were carried out and the results of the experiments are listed in Table C.
Of the experiments mentioned in Table C Experiments 10, 11, 15, 16, 20, 22, 24, 26, 28 and 30 are experiments according to the invention. In these experiments the Zr or Ti-promoted Co/SiO$_2$ catalysts which had been prepared by depositing cobalt and the promoter on the silica carrier by impregnation and/or kneading, were used for the production of hydrocarbons from H$_2$/CO mixtures containing 20—33% v of steam, calculated on the H$_2$/CO/H$_2$O mixture. In these experiments relative selectivity gains were obtained of 17—31%. The other experiments fall outside the scope of the invention. They have been included in the patent application for comparison. In Experiments 1—3 an iron catalyst was used; in Experiments 4—6 an unpromoted cobalt catalyst was used; in Experiments 7 and 8 a cobalt catalyst prepared substantially by precipitation was used. In Experiments 9, 12, 13, 19, 21, 23, 25, 27 and 29 the feed used was a H$_2$/CO mixture to which no steam had been added. In Experiments 14 and 17 the feed used was a H$_2$/CO mixture containing too little and too much steam, respectively. In these experiments no significant relative selectivity gain was obtained. In Experiment 18 the amount of steam was far too large, which resulted in a drop of C$_3$$^+$ selectivity.

Catalytic hydrotreatment
An Experiment 31 was carried out in which the C$_5$$^+$ fraction of the product obtained according to Experiment 26 was passed together with hydrogen through a 50-ml reactor containing a fixed catalyst bed, at a temperature of 345°C, a pressure of 130 bar, a space velocity of 1.25 l · l$^{-1}$ · h$^{-1}$ and a hydrogen/oil ratio of 2000 N1 · l$^{-1}$. The catalyst was a Pt/SiO$_2$—Al$_2$O$_3$ catalyst containing 0.82 parts by weight platinum per 100 pbw of carrier, which carrier consisted of 14.6% by weight of alumina and 85.4% by weight of silica. The results of Experiment 31 are given in Table D.

From the results given in Table D it appears that when a catalytic hydrotreatment is applied to a product prepared according to the invention, a considerable part of the $400°C^+$ fraction is converted (a decrease from 44 to 16% w) and a considerably quantity of $150—360°C$ fraction is formed (an increase from 36 to 58% w), whereas only very little $150°C^-$ fraction is fromed (an increase from 12 to 17% w).

TABLE B

| Catalyst No. | 4 | 5 | 6 | 7 | 8 |
|---|---|---|---|---|---|
| Cobalt load, mg cobalt per ml catalyst | 115 | 130 | 105 | 105 | 118 |
| Surface area, m² per ml catalyst | 22 | 15 | 30 | 28 | 21 |

TABLE C

| Experiment No. | 1 | 2 | 3 | 4 | 5 | 6 | 7 | 8 | 9 | 10 |
|---|---|---|---|---|---|---|---|---|---|---|
| Catalyst No. | 1 | 1 | 1 | · 2 | 2 | 2 | 3 | 3 | 4 | 4 |
| Temperature, °C | 250 | 250 | 249 | 250 | 252 | 253 | 250 | 255 | 250 | 250 |
| Pressure, bar | 30 | 30 | 30 | 30 | 30 | 30 | 30 | 30 | 30 | 30 |
| Space velocity, $1(H_2+CO) \cdot l^{-1} \cdot h^{-1}$ | 1000 | 1000 | 1000 | 1500 | 1500 | 1500 | 3000 | 3000 | 3000 | 3000 |
| $H_2/CO$ molar ratio of feed | 1 | 1 | 1 | 1 | 1 | 1 | 1 | 1 | 1 | 1 |
| $H_2O$ present in feed, % v on $(H_2+CO+H_2O)$ | 0 | 5 | 30 | 0 | 20 | 33 | 0 | 20 | 0 | 20 |
| CO conversion, % v | 39 | 8 | 5 | 35 | 35 | 34 | 32 | 31 | 37 | 35 |
| $C_3^+$ selectivity, calculated on $C_1^+$, % w | 87 | 71 | 67 | 84 | 84 | 84 | 83 | 81 | 85 | 89 |
| Relative selectivity gain, calculated on difference between theoretically attainable $C_3^+$ selectivity (100%) and actual $C_3^+$ selectivity without steam addition, % | — | * | * | — | 0 | 0 | — | * | — | 27 |

*selectivity decrease

0 109 702

TABLE C (contd.)

| Experiment No. | 11 | 12 | 13 | 14 | 15 | 16 | 17 | 18 | 19 | 20 |
|---|---|---|---|---|---|---|---|---|---|---|
| Catalyst No. | 4 | 4 | 5 | 5 | 5 | 5 | 5 | 5 | 5 | 5 |
| Temperature, °C | 252 | 252 | 219 | 220 | 221 | 222 | 224 | 224 | 238 | 240 |
| Pressure, bar | 30 | 30 | 20 | 20 | 20 | 20 | 20 | 20 | 20 | 20 |
| Space velocity, $1(H_2+CO) \cdot l^{-1} \cdot h^{-1}$ | 3000 | 3000 | 1200 | 1200 | 1200 | 1200 | 1200 | 1200 | 4000 | 4000 |
| $H_2/CO$ molar ratio of feed | 1 | 1 | 3 | 3 | 3 | 3 | 3 | 3 | 3 | 3 |
| $H_2O$ present in feed, % v on $(H_2+CO+J_2O)$ | 20 | 0 | 0 | 5 | 20 | 33 | 50 | 60 | 0 | 33 |
| CO conversion, % v | 37 | 39 | 79 | 80 | 80 | 79 | 80 | 78 | 59 | 60 |
| $C_3^+$ selectivity, calculated on $C_1^+$, % w | 88 | 84 | 82 | 83 | 85 | 86 | 83 | 78 | 63 | 73 |
| Relative selectivity gain, calculated on difference between theoretically attainable $C_3^+$ selectivity (100%) and actual $C_3^+$ selectivity without steam addition, % | 20 | — | — | 6 | 17 | 22 | 6 | * | — | 27 |

*selectivity decrease

TABLE C (contd.)

| Experiment No. | 21 | 22 | 23 | 24 | 25 | 26 | 27 | 28 | 29 | 30 |
|---|---|---|---|---|---|---|---|---|---|---|
| Catalyst No. | 5 | 5 | 6 | 6 | 7 | 7 | 7 | 7 | 8 | 8 |
| Temperature, °C | 250 | 253 | 204 | 205 | 205 | 207 | 221 | 222 | 248 | 251 |
| Pressure, bar | 30 | 30 | 20 | 20 | 20 | 20 | 20 | 20 | 30 | 30 |
| Space velocity, $1(H_2+CO) \cdot l^{-1} \cdot h^{-1}$ | 3000 | 3000 | 2000 | 2000 | 2000 | 2000 | 3000 | 3000 | 3000 | 3000 |
| $H_2/CO$ molar ratio of feed | 2 | 2 | 3 | 3 | 3 | 3 | 3 | 3 | 1 | 1 |
| $H_2O$ present in feed, % v on $(H_2+CO+H_2O)$ | 0 | 25 | 0 | 20 | 0 | 30 | 0 | 20 | 0 | 30 |
| CO conversion, % v | 65 | 65 | 60 | 60 | 71 | 72 | 81 | 81 | 38 | 39 |
| $C_3^+$ selectivity, calculated on $C_1^+$, % w | 78 | 83 | 87 | 90 | 87 | 91 | 80 | 85 | 84 | 87 |
| Relative selectivity gain, calculated on difference between theoretically attainable $C_3^+$ selectivity (100%) and actual $C_3^+$ selectivity without steam addition, % | — | 23 | — | 23 | — | 31 | — | 25 | — | 19 |

*selectivity decrease

TABLE D

| Composition, % w | $C_1^+$ product of Experiment 26 | $C_5^+$ fraction of the $C_1^+$ product of Experiment 26 | $C_1^+$ product after the catalytic hydrotreatment |
|---|---|---|---|
| $C_4^-$ | 16 | — | 2 |
| $C_5$—150°C | 10 | 12 | 15 |
| 150—250°C | 13 | 16 | 25 |
| 250—360°C | 17 | 20 | 33 |
| 360—400°C | 7 | 8 | 9 |
| 400°C$^+$ | 37 | 44 | 16 |

**Claims**

1. A process for the preparation of hydrocarbons by catalytic reaction of carbon monoxide with hydrogen, characterized in that a feed which comprises hydrogen, carbon monoxide and steam and in which the quantity of steam present is 10—40% v calculated on the $H_2/CO/H_2O$ mixture, is contacted at elevated temperature and pressure with a catalyst which comprises 5—40 pbw cobalt and 0.1—150 pbw of a promoter chosen from the group formed by zirconium, titanium or chromium per 100 pbw silica and has been prepared by depositing cobalt and the promoter on a silica carrier by impregnation and/or kneading and calcining and reducing the composition obtained, with the proviso that the catalyst does not contain an additional CO-shift catalyst.

2. Process as claimed in claim 1, characterized in that the catalyst comprises zirconium as a promoter.

3. A process as claimed in claim 1 or 2, characterized in that it is carried out at a temperature of 125—350°C and a pressure of 5—150 bar.

4. A process as claimed in any one of claims 1—3, characterized in that it is used as part of a multi-step process for the conversion of a $H_2$ and CO-containing feed.

5. A process as claimed in claim 4, characterized in that it is used as the second step of a two-step process in which light hydrocarbons, together with an excess of steam, are subjected in the first step to catalytic steam reforming and in which the $H_2/CO/H_2O$ mixture thus obtained is used as the feed for the second step.

6. A process as claimed in claim 4, characterized in that it is used as the second step of a two-step process in which a $H_2/CO$ mixture is contacted in the first step with a catalyst comprising one or more metal components with catalytic activity for converting a $H_2/CO$ mixture into hydrocarbons and/or oxygen-containing organic compounds and in which unconverted $H_2$ and CO present in the reaction product of the first step—together with other components of that reaction product, if desired—after the addition of steam, are used as the feed for the second step.

7. A process as claimed in claim 4, characterized in that it is used as the first step of a two-step process for preparing middle distillates from a $H_2$ and CO-containing feed, in which at least the part of the reaction product of the first step whose initial boiling point lies above the final boiling point of the heaviest middle distillate desired as end-product is subjected in the second step to a catalytic hydrotreatment.

8. A process as claimed in claim 4, characterized in that it is used as the second step of a three-step process for preparing, inter alia, middle distillates from a $H_2$ and CO-containing feed, in which the catalytic hydrotreatment mentioned in claim 7 is the third step applied to relevant heavy fraction of the reaction product of the second step of the two-step process mentioned in claims 5 and 6.

**Patentansprüche**

1. Verfahren zur Herstellung von Kohlenwasserstoffen durch katalytische Umsetzung von Kohlenmonoxid mit Wasserstoff, dadurch gekennzeichnet, daß ein Wasserstoff, Kohlenmonoxid und Dampf umfassendes Einsatzmaterial, worin die enthaltene Dampfmenge 10 bis 40 Vol.-%, bezogen auf das $H_2/CO/H_2O$-Gemisch beträgt, bei erhöhter Temperatur und bei erhöhtem Druck mit einem Katalysator in Berührung gebracht wird, der 5 bis 50 Gewichtsteile Kobalt und 0,1 bis 150 Gewichtsteile eines Promotors, ausgewählt aus der aus Zirkon, Titan und Chrom gebildeten Gruppe, je 100 Gewichtsteile Siliziumdioxid enthält und durch Ablagerung von Kobalt und dem Promotor auf einem Siliziumdioxidträger durch Imprägnieren und/oder Kneten und Kalzinieren und Reduzieren der erhaltenen Zusammensetzung erhalten

11

worden ist, mit der Maßgabe, daß der Katalysator keinen zusätzlichen CO-Verschiebungskatalysator enthält.

2. Verfahren nach Anspruch 1, dadurch gekennzeichnet, daß der Katalysator Zirkon als Promotor enthält.

3. Verfahren nach Anspruch 1 oder 2, dadurch gekennzeichnet, daß es bei einer Temperatur von 125 bis 350°C und bei einem Druck von 5 bis 150 bar ausgeführt wird.

4. Verfahren nach einem der Ansprüche 1 bis 3, dadurch gekennzeichnet, daß es als Teil eines Mehrstufenverfahrens für die Umwandlung eines Wasserstoff und Kohlenmonoxid enthaltenden Einsatzmaterials verwendet wird.

5. Verfahren nach Anspruch 4, dadurch gekennzeichnet, daß es als die zweite Stufe eines Zweistufenverfahrens verwendet wird, worin leichte Kohlenwasserstoffe zusammen mit einem Überschuß an Dampf in der ersten Stufe einer katalytischen Dampfreformierung unterworfen werden und worin das solcherart erhaltene $H_2/CO/H_2O$-Gemisch als Einsatzmaterial für die zweite Stufe verwendet wird.

6. Verfahren nach Anspruch 4, dadurch gekennzeichnet, daß es als die zweite Stufe eines Zweistufenverfahrens verwendet wird, worin ein $H_2/CO$-Gemisch in der ersten Stufe mit einem Katalysator in Berührung gebracht wird, der eine oder mehrere Metallkomponenten mit katalytischer Aktivität für die Umwandlung eines $H_2/CO$-Gemisches in Kohlenwasserstoffe und/oder Sauerstoff enthaltende organische Verbindungen umfaßt und worin im Reaktionsprodukt aus der ersten Stufe vorliegender nicht umgewandelter Wasserstoff und vorliegendes nicht umgewandeltes Kohlenmonoxid—gewünschtenfalls zusammen mit anderen Komponenten dieses Reaktionsproduktes—nach Zusatz von Dampf als Einsatzmaterial für die zweite Stufe verwendet werden.

7. Verfahren nach Anspruch 4, dadurch gekennzeichnet, daß es als die erste Stufe eines Zweistufenverfahrens zur Herstellung von Mitteldestillaten aus einem Wasserstoff und Kohlenmonoxid enthaltenden Einsatzmaterial verwendet wird, worin zumindest jener Teil des Reaktionsproduktes aus der ersten Stufe, dessen Anfangssiedepunkt oberhalb des Endsiedepunktes des als Endprodukt gewünschten schwersten Mitteldestillats liegt, in der zweiten Stufe einer katalytischen Wasserstoffbehandlung unterworfen wird.

8. Verfahren nach Anspruch 4, dadurch gekennzeichnet, daß es als die zweite Stufe eines Dreistufenverfahrens zur Herstellung von unter anderem Mitteldestillaten aus einem Wasserstoff und Kohlenmonoxid enthaltenden Einsatzmaterial verwendet wird, worin die in Anspruch 7 angeführte katalytische Wasserstoffbehandlung die dritte Stufe, angewendet auf die entsprechende schwere Fraktion des Reaktionsproduktes aus der zweiten Stufe des in den Ansprüchen 5 und 6 angeführten Zweistufenverfahrens, ist.

**Revendications**

1. Procédé de préparation d'hydrocarbures par réaction catalytique de monoxyde de carbone avec de l'hydrogène, caractérisé en ce qu'on met en contact une charge qui comprend de l'hydrogène, du monoxyde de carbone et de la vapeur et où la quantité de vapeur présente est de 10 à 40% en volume calculée par rapport au mélange $H_2/CO/H_2O$, à une température et une pression élevées avec un catalyseur qui comprend 5—40% en poids de cobalt et 0,1—150% en poids d'un promoteur choisi dans le groupe formé par le zirconium, le titane ou le chrome pour 100 parties en poids de silice et qui a été préparé par dépôt de cobalt et du promoteur sur un support de silice par imprégnation et/ou pétrissage et calcination et réduction de la composition obtenue, avec cette condition que le catalyseur ne contienne pas de catalyseur de déplacement de CO supplémentaire.

2. Procédé selon la revendication 1, caractérisé en ce que le catalyseur comprend du zirconium comme promoteur.

3. Procédé selon la revendication 1 ou 2, caractérisé en ce qu'il est conduit à une température de 125—350°C et une pression de 5—150 bar.

4. Procédé selon l'une quelconque des revendications 1—3, caractérisé en ce qu'il est utilisé comme partie d'un procédé multi-étape pour la transformation d'une charge contenant $H_2$ et CO.

5. Procédé selon la revendication 4, caractérisé en ce qu'il est utilisé comme seconde étape d'un procédé à deux étapes dans lequel on soumet des hydrocarbures légers, avec un excès de vapeur, dans la première étape à un réformage à la vapeur catalytique et où le mélange $H_2/CO/H_2O$ ainsi obtenu est utilisé comme charge pour la seconde étape.

6. Procédé selon la revendication 4, caractérisé en ce qu'il est utilisé comme seconde étape d'un procédé à deux étapes où l'on met en contact un mélange $H_2/CO$ dans la première étape avec un catalyseur contenant un ou plusieurs composants métalliques ayant une activité catalytique pour transformer un mélange $H_2/CO$ an hydrocarbures et/ou en composés organiques contenant de l'oxygène et où $H_2$ et CO non transformés présents dans le produit réactionnel de la première étape -avec d'autres composants de ce produit réactionnel, si on le désire-après l'addition de vapeur, sont utilisés comme charge pour la seconde étape.

7. Procédé selon la revendication 4, caractérisé en ce qu'il est utilisé comme première étape d'un procédé en deux étapes pour préparer des distillats moyens à partir d'une charge contenant $H_2$ et CO, où au moins une partie du produit réactionnel de la première étape dont le point d'ébullition initiale se situe audessus du point d'ébullition final du distillat moyen le plus lourd désiré comme produit final et soumise à dans la seconde étape à un hydrotraitement catalytique.

8. Procédé selon la revendication 4, caractérisé en ce qu'il est utilisé comme seconde étape d'un procédé en trois étapes pour préparer, entre autre, des distillats moyens à partir d'une charge contenant $H_2$ et CO, où l'hydrotraitement catalytique mentionné dans la revendication 7 est la troisième étape appliquée à la fraction lourde concernée du produit réactionnel de la seconde étape du procédé en deux étapes mentionné dans les revendications 5 et 6.